(19) Europäisches Patentamt
European Patent Office
Office européen des brevets

(11) **EP 1 726 659 B1**

(12) **EUROPEAN PATENT SPECIFICATION**

(45) Date of publication and mention
of the grant of the patent:
**30.12.2009 Bulletin 2009/53**

(51) Int Cl.:
*A61K 38/04* (2006.01)    *A61K 38/05* (2006.01)

(21) Application number: **05104422.0**

(22) Date of filing: **24.05.2005**

(54) **Isolated dipeptides with ACE inhibitor effect**

Isolierte Dipeptide mit ACE-Inhibitionseffekt

Dipeptides isolés avec effet ACE-inhibiteur

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(43) Date of publication of application:
**29.11.2006 Bulletin 2006/48**

(73) Proprietor: **Chr. Hansen A/S**
**2970 Hoersholm (DK)**

(72) Inventor: **Flambard, Benedicte**
**1964, Frederiksberg (DK)**

(56) References cited:
**EP-A- 1 231 279     WO-A-2004/082709**

• **LULY J R ET AL: "NEW INHIBITORS OF HUMAN RENIN THAT CONTAIN NOVEL LEU-VAL REPLACEMENTS" JOURNAL OF MEDICINAL CHEMISTRY, AMERICAN CHEMICAL SOCIETY. WASHINGTON, US, vol. 30, no. 9, 1987, pages 1609-1616, XP001053395 ISSN: 0022-2623**
• **MATSUI T ET AL: "DEPRESSOR EFFECT OF WHEAT GERM HYDROLYSATE AND ITS NOVEL ANGIOTENSIN I-CONVERTING ENZYME INHIBITORY PEPTIDE, IIE-VAL-TYR, AND THE METABOLISM IN RAT AND HUMAN PLASMA" BIOLOGICAL & PHARMACEUTICAL BULLETIN (OF JAPAN), PHARMACEUTICAL SOCIETY OF JAPAN, TOKYO, JP, vol. 23, no. 4, April 2000 (2000-04), pages 427-431, XP008048155 ISSN: 0918-6158**

SEQUENCE LISTING

<110> Chr. Hansen A/S

<120> Isolated peptides with ACE inhibitor effect

<130> P2077EF00

<160> 2

<170> PatentIn version 3.1

<210> 1
<211> 2
<212> PRT
<213> Artificial

<400> 1

Leu Val
1

<210> 2
<211> 2
<212> PRT
<213> Artificial

<400> 2

Ile Val
1

## Description

### Field of Invention:

The present invention relates to the use of a preparation comprising an isolated peptide selected from the group consisting of: Leu-Val (LV) and Ile-Val (IV) for the preparation of a composition having angiotensin-converting enzyme (ACE) inhibitor effect. The peptides may e.g. be used to make a functional food with anti-hypertensive properties.

### Description of the Background Art:

Hypertension (high blood pressure) has been reported to be one of the most important risk factors associated with heart attack in industrialized countries. Hypertension is frequently treated with drugs that strongly inhibit the angiotensin-converting enzyme (ACE). The prevention of high blood pressure in the early stage of the development of the disease can be an alternative to the treatment of hypertension with drugs. A large number of food-derived bioactive compounds are currently considered beneficial for general well being or health promoting.

In the regulation of blood pressure, angiotensin I-converting enzyme (ACE) plays an important role. ACE acts to increase the blood pressure. In the renin-angiotensin system, ACE converts angiotensin-I to angiotensin-II by hydrolyzing His-Leu from its C-terminal. Angiotensin II exhibits a strong vasoconstricting action. Additionally, in the kinin kallikrein system, ACE deactivates bradykinin, which aids vasodilation. ACE inhibitors are therefore useful in reducing blood pressure. Currently, several ACE inhibitors already exist. The first reported ACE inhibitors were naturally occurring peptides found in snake venom. Since then, many other ACE inhibitors have also been discovered.

It is known that milk fermented by lactic acid bacteria (LAB) may produce anti-hypertensive effects due to the liberation of peptides from casein in the milk by the proteolytic activity of the lactic acid bacteria. The peptides act as ACE inhibitors.

EP583074-B1 (Calpis Ltd) describes isolated peptides with anti-hypertensive effects derived from LAB milk fermentation, wherein the peptides contain 3 to 10 amino acid residues and include the sequence Val-Pro-Pro (VPP). The peptides are isolated from the whey fraction of the fermented milk.

EP821968-A (Calpis Ltd) describes isolated peptides with anti-hypertensive effects derived from LAB milk fermentation, wherein the peptides contain a dipeptide Tyr-Pro (YP). The peptides are isolated from the whey fraction of the fermented milk.

EP1016709-A (Calpis Ltd) describes isolated peptides with anti-hypertensive effects derived from LAB milk fermentation, wherein the peptides include the sequence Ile-Pro-Pro (IPP). The peptides are isolated from the whey fraction of the fermented milk.

W004/098310 (Unilever) describes isolated peptides with anti-hypertensive effects derived from enzymatic hydrolyzation of casein, wherein the peptides are the pentapeptide ILAEK, the pentapeptide IPAVF and the hexapeptide IPAVFK.

The article (Yamamoto et al; Current Pharmaceutical design, 2003, 9, p1345-1355) with the title "Biogen Peptides and Their Potential Use) provides a review of different peptides derived from casein hydrolyzation (both enzymatic and by LAB fermentation). In table 2, page 1347 an overview of such known peptides is given. Besides the ones discussed above, table 2 mentions the following peptides: FFVAPFPEVFGK, FFVAP, VAP, FVAP, TTMPLW, PLW, LW, YKVPQL, FP, TPVVVPPFLQP, AVPYPQR, VYPFPG, VYP, IPA, VFK, LAMA, LDAQSAPLR, ALPMH.

WO2004/015125 (Chr. Hansen A/S) describes a special LAB cell (*Lactobacillus helveticus* bacterium with the registration number DSM 14998), which can be used to make a fermented milk product with very high anti-hypertensive effects. The LAB with registration number DSM 14998 may herein also be termed Cardi04 or CHCC5951. The described LAB is characterized in that it comprises some particular specific cell wall proteases (prtH200 and orfF3). The theory is that these proteases give a specific hydrolyzation profile of milk casein and thereby a specific profile of different peptides with anti-hypertensive effects resulting in fermentation milk products with very high anti-hypertensive effects.

Table 4 (page 55) of WO2004/015125 demonstrates that CHCC5951 whole fermented milk has a significant improved anti-hypertensive effects compared to milk fermented with other LAB strains. In fact, the effect was comparable to the effect of Enalapril (Enalapril is a medicine usually used to treat patients with high blood pressure).

As explained, WO2004/015125 describes fermented milk as such (whole fermented milk), which has been fermented with the special DSM 14998 LAB strain. However, WO2004/015125 does not explicitly describe special peptides isolated from the whole fermented milk.

### Summary of invention:

The problem to be solved by the present invention is to provide novel peptides having angiotensin-converting enzyme (ACE) inhibitor effect. The peptides may e.g. be used to make a functional food with anti-hypertensive properties.

The solution is based on the present inventors having further analyzed fermented milk, which has been fermented with the special DSM 14998 LAB strain as described in WO2004/015125. Based on this analysis novel peptides with surprisingly good anti-hypertensive properties were identified.

After centrifugation, whole fermented milk can be separated into the supernatant fraction (whey fraction) and the precipitate fraction (herein termed "fat+cell fraction"). The novel peptides described herein were identified in the fat+cell fraction. This is surprising since the prior art generally teaches that relevant peptides with anti-hypertensive properties are normally found in the whey (see background section above). For further details, please see working examples herein.

Accordingly, a first aspect of the invention relates to the use of a preparation comprising an isolated peptide selected from the group consisting of: Leu-Val (LV) and Ile-Val (IV) for the preparation of a composition having angiotensin-converting enzyme (ACE) inhibitor effect.

The peptide Leu-Val (LV) is shown as SEQ ID NO 1 herein.
The peptide Ile-Val (IV) is shown as SEQ ID NO 2 herein.

Advantages of the herein described peptides are that they are relatively hydrophobic (preferential substrates of the ACE has been reported hydrophobic) and small (can cross the intestinal barrier easily). Further, since they are small they are easy to make synthetically.

**Definitions:**

Prior to a discussion of the detailed embodiments of the invention, a definition of specific terms related to the main aspects of the invention is provided.

The term "isolated" within the sentence "a preparation comprising an isolated peptide" denotes herein a relatively pure preparation of the peptide of interest. For instance, a preparation wherein at least 10% (w/w) of the total peptide content of the preparation is the peptide of interest, more preferably wherein at least 50% (w/w) of the total peptide content of the preparation is the peptide of interest and most preferably wherein at least 90% (w/w) of the total peptide content of the preparation is the peptide of interest. In the present context, the term "isolated" may also be seen as some relevant purification of the peptide as described herein from the other peptides of a LAB fermented milk. The preparation may comprise one or more of the peptides of the first aspect of the invention, i.e. it may be a mixture of one or more of the peptides of the first aspect of the invention. If the preparation is a mixture comprising more than one of the peptides of the first aspect, then the w/w % is calculated as the sum of the w/w % of the individual peptides.

Embodiment(s) of the present invention is described below, by way of example(s) only.

**Detailed description of the invention:**

Peptides of the invention:

The peptides of the first aspect of the invention may be obtained by fermenting milk casein with the *Lactobacillus helveticus* bacterium with the registration number DSM 14998 described in W02004/015125, centrifugating the fermented milk and separating it into a supernatant fraction (whey fraction) and a precipitate fraction (herein termed "fat+cell fraction"), making an aqueous extract of the fat+cell fraction and isolating the relevant peptides from this aqueous extract. For further details reference is made to working examples herein.

Alternatively, the peptides may be made synthetically according to methods known in the art.

The preparation comprising the isolated peptides of interest may be in any suitable form such a in a dried form, preferably as in a freeze-dried form.

In order to comprise sufficient amount of peptide of interest for a relevant industrial use the preparation should preferably have a certain total weight. Preferably, the preparation has a total weight of from 10g to 100kg, more preferably from 100g to 100 kg.

Composition having angiotensin-converting enzyme (ACE) inhibitor effect

The composition having angiotensin-converting enzyme (ACE) inhibitor effect may be in any suitable form. Essentially it will depend on the relevant use of the composition.

If e.g. the composition is used as a food additive suitable to be added to a relevant food product to make e.g. a functional food product, then the food additive composition may preferably be in a dried form such as in a freeze-dried form.

In a preferred embodiment, the preparation comprising the isolated peptides as described herein is added to a food product to make a functional food product. In such a case the composition having angiotensin-converting enzyme (ACE) inhibitor effect, of the first aspect, is a functional food product.

The amount of peptides added to the food product will generally vary depending on the specific food product of interest. However, generally speaking it is preferred that a food product comprises from 0.01 to 15 wt% of the peptides as described herein, more preferably from 0.1 to 10 wt% of the peptides as described herein.

The food products may be of any food type. They may comprise common food ingredients in addition to the food product, such as flour, sugar, fruits, minerals, vitamins, stabilizers, thickeners, etc. in appropriate amounts.

Preferably, the food products are fruit juice products, dairy type products, frozen confectionary products or spreads/margarines. These preferred types of food products are described in some detail below.

Examples of fruit juice products are juices derived from citrus fruit like orange and grapefruit, tropical fruits, banana, peach, peer or strawberry.

Examples of dairy products are milk, dairy spreads, cream cheese, milk type drinks and yoghurt. The food product may be a milk type drink.

In the present context, frozen confectionery product includes milk containing frozen confections such as ice-cream, frozen yoghurt, sherbet, sorbet, ice milk and frozen custard, water-ices, granitas or frozen fruit purees.

Alternatively, the composition having angiotensin-converting enzyme (ACE) inhibitor effect, of the first aspect, is a medicament.

When the peptides as described herein are used in a medicament, a preferred effective amount of the peptides as described herein varies depending upon the age and condition of a person. Preferably it is in a range of 0.05 to 10mg/kg body weight/day.

Independently of the specific form (e.g. a food product or a medicament) the composition having angiotensin-converting enzyme (ACE) inhibitor effect, of the first aspect of the invention, may be used for treatment of different clinical relevant symptoms or diseases.

Accordingly, in a preferred embodiment the composition having angiotensin-converting enzyme (ACE) inhibitor effect is used for treatment of hypertension in a mammal, preferably a human.

In another embodiment, the composition having angiotensin-converting enzyme (ACE) inhibitor effect is used for reducing the heart rate in a mammal (preferably a human), in particular wherein it is done for treatment or relief of a coronary artery disease (CAD) or a coronary heart disease (CHD), such as angina pectoris, hypertension, atherosclerosis, stroke, myocardial infarction, cerebral infarction, and restenosis following angioplasty, arrhythmia, tachyarrhythmia, congestive heart failure (CHF), aortic valve regurgitation, chronic renal failure, dyslipidemia, dyslipoproteinemia.

The art describes that ACE inhibition drugs (Enalapril) may have a positive effect in a cholesterol lowering therapy in particular in reducing coronary atherosclerotic disease.

Accordingly, an embodiment of the invention is wherein the composition having angiotensin-converting enzyme (ACE) inhibitor effect is used for a cholesterol lowering therapy in particular in relation to reducing coronary atherosclerotic disease.

## EXAMPLES:

MATERIAL AND METHODS:

Fermentation of milk:

Fermentation of milk with the *Lactobacillus helveticus* bacterium with the registration number DSM 14998 was done essentially as described in WO2004/015125.

Isolation of peptides with ACE activity

Isolation of the peptides and analysis of these for ACE activity was done based on a socalled BioSelect™ protocol. This protocol is described in details in WO00/65353, WO00/65354 and W00237111. Below relevant elements of the protocol are outlined after.

BioSelact™ based protocol

*Sample preparation*

Supernatants of whole fermentations, whey liquids, chloroform and aqueous extracts from fat/cell fractions were all pH adjusted before analysis. First the solutions were adjusted to pH 3 (*pH of LC solutions).* After centrifugation the precipitate was removed. During a second step the pH was adjusted to 7.5 (pH of bioassay). Again precipitate was removed from the solution after centrifugation. The remaining supernatants were used for BioSelact™ analysis.

*Extraction*

Aqueous extract fat/cell preparation

250 mg of whey fat/cell preparation was extracted with 250 µl MilliQ. The solution was positioned on a flat shaker for 2 hours at room temperature. The fat/cell fraction was removed after extraction by centrifugation.

Organic extract fat/cell preparation

1.5 g of whey fat/cell preparation was extracted with chloroform. The solution was positioned on a flat shaker for 2h at room temperature. The fat/cell fraction was removed after extraction. Next, chloroform was evaporated under nitrogen at room temperature. The pellet was dissolved in 75 µl methanol and diluted with MilliQ to a 30% MeOH solution.

*BioSelact™ analysis*

During the first step of the BioSelact™ analysis, the aqueous solutions and chloroform extracts of various parts of the whey preparations were analyzed by applying a general chromatographic gradient: 5 - 95% MeOH/0.05% TFA in 75 min. For these general analyses a Phenomenex, LUNA C18(2), 5 µm, 2* 250 mm analytical column was used. The chromatographic separation was carried out at a flow rate of 0.2 ml/min at ambient temperature. The injection volume was 250 µl. The organic modifier concentration in the LC effluent was decreased to bioassay compatible levels by performing continuous dilution using the BioGradient from the company Kiadis. In this case the output of the BioGradient resulted in a constant effluent flow rate of 1 ml/min and a constant modifier percentage of 10% MeOH. Part of the effluent solution was mixed on-line with biochemical reagents to perform the ACE bioassay. In addition, an equal flow rate was directed in parallel towards a reference assay in order to measure the presence of autofluorescence and eliminate false positive results. In addition, part of the LC effluent was introduced on-line into a MS detector in order to obtain chemical information of the bioactive compounds.
After completing the first general chromatographic 'sweep', the presence of bioactivity in each sample was carefully assessed. In case of co-elution 'tailor-made' chromatographic gradients were performed in order to enhance resolution and enable accurate mass assignment. For these purposes several types of 2 * 250 mm, 5 µm, C18 Vydac columns were used.

*Mass spectrometry*

A QTOF-micro MS detector *(Waters, Holland)* was used during the entire project. The MS detector was calibrated daily using a 0.1% phosphoric acid solution. Sensitivity specifications were monitored daily as well. Erythromycin was used as a reference mass and was introduced continuously into the MS detector via a lock-spray. In order to enhance ionization a MeOH/2% acetic acid solution was added to the LC effluent just before being sprayed into the MS detector. During 'routine' measurements the MS detector was operated in full scanning positive ion electrospray ionization mode *(ESI pos)*. For identity confirmation purposes the MS was operated in MS/MS positive ESI mode. During these measurements a collision energy of 25 kV was typically used.

*Accurate mass assignment*

During each screening session a reference sample, containing the reference inhibitors IPP and VPP, was analyzed in order to determine the correlation time between the biochemical (bioassay) and chemical data (MS). This correlation time is constant and allows one to compensate for the time difference in actually measuring a particular compound in the bioassay and MS detector. Subsequently, candidate masses that correlate with the bioactive compound are searched. Especially in complex samples, such as whey fermentations, multiple candidates can be found for a single active compound. In these cases additional separations are applied to reduce the number of mass candidates and enable accurate mass assignment.

*Peptide identification*

During a first step, the accurate masses of the bioactive compounds are used as search queries in a suitable ACE inhibitor database. If matches are found, the MS/MS data is used to confirm the identity of the substances. When no database matches are found, however, the accurate masses are used to find peptide sequences in the source milk proteins with similar molecular mass. Subsequently, the correct peptide sequence is selected by assessing the MS/MS spectra of the bioactive compound. In case no candidate sequences can be found that match the measured accurate mass, the MS/MS spectrum of the compound is fully interpreted.

*Sample pre-concentration*

In order to increase the concentration of bioactive compounds injected into the BioSelact™ platform, a straightforward on-line sample pre-concentration routine was used. During the first step sample volumes up to 1.2 ml were injected onto a LUNA precolumn (Mercury MS, Phenomenex). Subsequently, the column was washed and switched into the HPLC system directly afterwards.

Alternative ACE assay:

As an alternative to the BioSelact™ based protocol ACE activity assay as described herein, the ACE inhibitor effect of the peptides may be analyzed by use of the *in vitro* assay described below.

*ACE activity assay*

The peptides of interest are tested for ACE activity *in vitro.* The DL50 (mg/ml) is the peptidic concentration, which inhibits 50% of ACE activity. The lower this value is, the better the anti-hypertensive effect of the fermented milk. The ACE activity of the peptides is measured by the following protocol:

The essence of the assay is that ACE degrades a hippuryl-L-histidyl-L-leucine (HHL) substrate and adding a color agent develops a color. If peptides are present the peptides inhibit ACE and less HHL substrate is degraded. This means less color is developed after addition of the color agent.

Solution preparation:

Incubation buffer: 188 mmol/l boric acid pH 8.3, 1.375 mmol/l potassium chloride.
(Dissolve 2.91 g of boric acid and 25.63 g potassium chloride in 200 ml of distilled water. Adjust the pH to 8.3 with 1 mol/l potassium hydroxide and dilute to 250 ml with distilled water. Store at room temperature).
Substrate solution: 5.8 mmol/l hippuryl-L-histidyl-L-leucine (HHL).
(Dissolve 250 mg hippuryl-L-histidyl-L-leucine in about 90 ml incubation buffer and fill up to 100 ml with the same buffer. Store at 40˚C. The substrate solution can be used for at least 2 weeks).
Stop solution: 100 mmol/l HEPES pH 9, 2.5 mmol EDTA.
(Dissolve 23.83 g HEPES and 0.93 g EDTA in 800 ml distilled water. Adjust to pH 9 with 1 mol/l sodium hydroxide and dilute to 1 1 with distilled water. Store at room temperature).
Color reagent: 136 mmol/l cyanuric chloride in 1, 4-dioxane.
(Dissolve 12.50 g cyanuric chloride in about 400 ml of 1, 4-dioxane and fill up with 1,4-dioxane to 500 ml. Store at room temperature in dark-brown glass bottle).
Assay: (all solutions are equilibrated to room temperature)

- Make a dilution series of the peptide solution with incubation buffer. The series consist of 6 dilutions going from the undiluted peptide solution to a blank (only incubation buffer)
- For each of the dilutions, place 10 μl of peptide solution, 40 μl of substrate (HHL) solution (2.5 g/l) and 2.5 μl of ACE (0.25 Units/ml) in a glass tube.
- The positive control comprises 2.5 μl ACE, 10 μl of incubation and 40 μl of substrate (HHL)
- The negative control comprises 12 μl of incubation buffer and 40 μl of substrate (HHL)
- Incubate at 37˚C for 1 hour.
- Stop the reaction by adding 300 μl of stop solution, followed by 150 μl of color reagent - Mix vigorously.
- Allow to stand for 5 minutes and centrifuge at 3300 g for 30 min at room temperature to remove denatured protein and excess cyanuric chloride.
- Transfer 300 μl of supernatant of each sample to microtiter plate hole.
- Read at 405 nm against water as a blank.

The ACE inhibition percentage is expressed by the formula;

$$\text{ACE inhibition activity} = \frac{\text{OD405nm positive control} - \text{OD405nm sample}}{\text{OD405nm positive control} - \text{OD405nm negative control}}$$

Each dilution has its own ACE inhibition percentage value that gives a curve expressing the ACE inhibition percentage in function of the peptide concentration of the whey. DL50 (peptidic concentration that inhibits 50 % of ACE activity) is obtained by reading the peptidic concentration at the intersection point between the curve and the corresponding 50 % ACE inhibition point on the axis.

EXAMPLE _1: ACE inhibitor effect of the isolated LV and IV di-peptides._

The LV and IV di-peptides were isolated from an aqueous fat/cell extract based on the Bioselact™ based protocol as described above.
The ACE inhibitor activity was measured and it was found that both the LV and IV peptides had a significant better angiotensin-converting enzyme (ACE) inhibitor effect than other peptides isolated from the aqueous fat/cell extract.

EXAMPLE 2: _Comparison of peptides derivable from fermentation of two different LAB strains._

The fermentation of milk as described above was repeated by use of a different LAB strain. The different LAB strain was a commercial relevant JM1004 strain from Calpis (Japan).
The results demonstrated that an aqueous fat/cell extract derived from milk fermented with the JM1004 LAB strain did not comprise the herein described LV and IV peptides.
Without being limited to theory, this may be one possible explanation of the fact that milk fermented with the LAB strain _Lactobacillus helveticus_ bacterium with the registration number DSM 14998 (described in W02004/015125) generally has an improved (ACE) inhibitor effect as compared to milk fermented with other comparable LAB strains.

**REFERENCES:**

Below are mentioned references that are considered relevant in relation to the present invention.

EP583074-B1 (Calpis Ltd)

EP821968-A (Calpis Ltd)

EP1016709-A (Calpis Ltd)

W004/098310 (Unilever)

Yamamoto et al; Current Pharmaceutical design, 2003, 9, p1345-1355

WO2004/015125 (Chr. Hansen A/S)

WO00/65353

WO00/65354

W00237111.

## Claims

1. Use of a preparation comprising an isolated peptide selected from the group consisting of: Leu-Val (LV) and Ile-Val (IV) for the preparation of a composition having angiotensin-converting enzyme (ACE) inhibitor effect.

2. The use of claim 1, wherein the preparation is a preparation wherein at least 50% (w/w) of the total peptide content of the preparation is a peptide selected from the group of claim 1.

3. The use of claims 1 or 2, wherein the composition having angiotensin-converting enzyme (ACE) inhibitor effect is a functional food product.

4. The use of claim 3, wherein the functional food product is a dairy type product, such as milk, dairy spreads, cream cheese, milk type drinks and yoghurt.

5. The use of claims 1 or 2, wherein the composition having angiotensin-converting enzyme (ACE) inhibitor effect is a medicament.

6. The use of any of claims 1 to 5, wherein the composition having angiotensin-converting enzyme (ACE) inhibitor effect is used for treatment of hypertension in a human.

7. The use of any of claims 1 to 5, wherein the composition having angiotensin-converting enzyme (ACE) inhibitor effect is used for reducing the heart rate in a human, in particular wherein it is done for treatment or relief of a coronary artery disease (CAD) or a coronary heart disease (CHD), such as angina pectoris, hypertension, atherosclerosis, stroke, myocardial infarction, cerebral infarction, and restenosis following angioplasty, arrhythmia, tachyarrythmia, congestive heart failure (CHF), aortic valve regurgitation, chronic renal failure, dyslipidemia, dyslipoproteinemia.

8. The use of any of claims 1 to 5, wherein the composition having angiotensin-converting enzyme (ACE) inhibitor effect is used for a cholesterol lowering therapy in a human, in particular in relation to reducing coronary atherosclerotic disease.

**Patentansprüche**

1. Verwendung eines Präparats mit einem isolierten Peptid ausgewählt aus der Gruppe bestehend aus: Leu-Val (LV) und Ile-Val (IV) zur Herstellung einer Zusammensetzung mit ACE (Angiotensin umwandelndes Enzym)-Hemmer-Wirkung.

2. Verwendung nach Anspruch 1, bei dem das Präparat ein Präparat ist, bei dem mindestens 50% (Gew./Gew.) des gesamten Peptidgehalts des Präparats ein Peptid aus der Gruppe nach Anspruch 1 ist.

3. Verwendung nach Anspruch 1 oder 2, wobei die Zusammensetzung mit ACE-Hemmer-Wirkung ein Lebensmittel mit definierten Verwendungseigenschaften ist.

4. Verwendung nach Anspruch 3, wobei das Lebensmittel mit definierten Verwendungseigenschaften ein Molkereiprodukt ist, wie zum Beispiel Milch, Milchstreichfette, Rahmkäse, Milchgetränke und Joghurt.

5. Verwendung nach Patentanspruch 1 oder 2, wobei die Zusammensetzung mit ACE-Hemmer-Wirkung ein Medikament ist.

6. Verwendung nach einem der Ansprüche 1 bis 5, wobei die Zusammensetzung mit ACE-Hemmer-Wirkung zur Behandlung von Bluthochdruck beim Menschen benutzt wird.

7. Verwendung nach einem der Ansprüche 1 bis 5, wobei die Zusammensetzung mit ACE-Hemmer-Wirkung benutzt wird um die Herzschlagfrequenz beim Menschen zu reduzieren, insbesondere zur Behandlung oder Linderung einer koronaren Arterienkrankheit (CAD) oder einer koronaren Herzerkrankung (CHD), wie zum Beispiel Angina pectoris, Bluthochdruck, Gefäßverkalkung, Schlaganfall, Herzmuskelinfarkt, Hirnschlag und Restenose nach Angioplastie, Herzrhythmusstörung, Vorhofflimmern, Stauungsinsuffizienz (CHF), Aortenklappeninsuffizienz, chronisches Nierenversagen, Dyslipidämie, Dyslipoproteinämie.

8. Verwendung nach einem der Ansprüche 1 bis 5, wobei die Zusammensetzung mit ACE-Hemmer-Wirkung für eine cholesterinsenkende Therapie beim Menschen benutzt wird, insbesondere bei Arterienverkalkung.

**Revendications**

1. Utilisation d'une préparation comprenant un peptide isolé sélectionné du groupe composé de : Leu-Val (LV) et Ile-Val (IV) pour la préparation d'une composition ayant un effet inhibiteur de l'enzyme de conversion de l'angiotensine (ECA).

2. L'utilisation de la revendication 1, dans laquelle la préparation est une préparation dans laquelle au moins 50% (m/m) du contenu de peptides total de la préparation est un peptide sélectionné du groupe de la revendication 1.

3. L'utilisation des revendications 1 ou 2, dans laquelle la composition ayant un effet inhibiteur de l'enzyme de conversion de l'angiotensine (ECA) est un produit alimentaire fonctionnel.

4. L'utilisation de la revendication 3, dans laquelle le produit alimentaire fonctionnel est un produit de type laitier, comme lait, produits à tartiner à base de lait, fromage blanc, boissons du type lait et yaourt.

5. L'utilisation des revendications 1 ou 2, dans laquelle la composition ayant un effet inhibiteur de l'enzyme de conversion de l'angiotensine (ECA) est un médicament.

6. L'utilisation de l'une quelconque des revendications 1 à 5, dans laquelle la composition ayant un effet inhibiteur de l'enzyme de conversion de l'angiotensine (ECA) est utilisée pour le traitement de l'hypertension chez l'être humain.

7. L'utilisation de l'une quelconque des revendications 1 à 5, dans laquelle la composition ayant un effet inhibiteur de l'enzyme de conversion de l'angiotensine (ECA) est utilisée pour réduire le rythme cardiaque chez l'être humain, en particulier pour le traitement ou le soulagement d'une maladie des artères coronaires (CAD) ou d'une coronaropathie (CHD), comme angine de poitrine, hypertension, athérosclérose, apoplexie, infarctus du myocarde, infarctus cérébral, et resténose après une angioplastie, arythmie, tachyarrythmie, insuffisance cardiaque (IC), régurgitation de la valve aortique, défaillance chronique de la fonction rénale, dyslipidémie, dyslipoprotéinémie.

8. L'utilisation de l'une quelconque des revendications 1 à 5, dans laquelle la composition ayant un effet inhibiteur de l'enzyme de conversion de l'angiotensine (ECA) est utilisée pour une thérapie de réduction de cholestérol chez l'être humain, en particulier en relation avec la diminution des maladies coronaires athérosclérotiques.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 583074 B1
- EP 821968 A
- EP 1016709 A
- WO 04098310 A
- WO 2004015125 A
- WO 0065353 A
- WO 0065354 A
- WO 0237111 A

**Non-patent literature cited in the description**

- **Yamamoto et al.** *Current Pharmaceutical design,* 2003, vol. 9, 1345-1355